# EUROPÄISCHE PATENTSCHRIFT

(11) **EP 0 337 486 B1**
(45) Veröffentlichungstag und Bekanntmachung des Hinweises auf die Patenterteilung: **19.07.1995**
(21) Anmeldenummer: 89106733.2
(22) Anmeldetag: 14.04.1989
(51) Int. Cl.: C12N 1/00, C12N 1/14, A23K 1/00, C12N 1/22

(54) **Verfahren zur Herstellung von Rübenpressschnitzeln durch Fermentation**
Method for the production of beet press cake by fermentation
Procédé de préparation de gâteaux pressés de betterares par fermentation

(30) Priorität: 15.04.1988 DE 3812612
(43) Veröffentlichungstag der Anmeldung: 18.10.1989
(73) Patentinhaber: SÜDZUCKER AKTIENGESELLSCHAFT MANNHEIM/OCHSENFURT, D-68165 Mannheim (DE)
(72) Erfinder: Nigam, Poonam, Dr., Kanpur 208001 (IN); Vogel, Manfred, Dr., D-6718 Grünstadt (DE)
(74) Vertreter: Gleiss, Alf-Olav, Dr.jur. Dipl.-Ing.

(56) Entgegenhaltungen:
- EP-A- 0 140 723
- EP-A- 0 225 479
- BE-A- 675 878
- BIOTECHNOLOGY & BIOENGINEERING, Band 30,Nr. 2, 5. August 1987, Seiten 282-288, John Wiley & Sons, Inc., New York, NY, US; U.E. VIESTURS et al.: "Combined submerged and solid substrate fermentation for the bioconversion of lignocellulose"

## Beschreibung

Die Erfindung betrifft ein Verfahren zur Herstellung von Preßschnitzeln gemäß dem Oberbegriff des Anspruchs 1. Insbesondere betrifft die Erfindung ein Verfahren zur Herstellung von proteinangereicherten Preßschnitzeln, indem man auf extrahierten Preßschnitzeln aus der Rübenzuckerfabrikation unter den erfindungsgemäßen Bedingungen einen niederen Pilz (fungi imperfecti) wachsen läßt. Das erhaltene Fermentationsprodukt kann nach einem Trocknungsprozeß als eiweißreiches Futter für monogastrische Tiere verwendet werden.
Extrahierte Rübenschnitzel fallen bei der Zuckergewinnung aus Zuckerrüben in großen Mengen an. Sie stellen die Gerüstsubstanz der Zuckerrübe dar und bestehen vorwiegend aus Hemicellulose, Cellulose und Pektin. Da solche Substanzen nur von Wiederkäuern verwertet werden können, werden extrahierte Rübenschnitzel entweder nach Abpressen als sogenannte Preßschnitzel mit einem Trockensubstanzgehalt von 20 - 35 % dazu verwendet oder die Preßschnitzel werden direkt oder nach Zugabe von Rübenmelasse auf 90 - 95 % Trockensubstanzgehalt getrocknet.
Nach weiteren Verwendungsmöglichkeiten von Preßschnitzeln ist in den letzten Jahren intensiv geforscht worden. So hat man sich u. a. mit der Biokonversion zu Biogas beschäftigt (K. Buchholz, E. Stoppok, R. Emmerich und U. Bartz, Zuckerindustrie 112, 7, 605 - 611, 1987). Andere haben die enzymatische Verflüssigung und Verzuckerung dieser Biomasse untersucht bzw. Preßschnitzel als Substrat für Fermentationen eingesetzt, um so Hydrolasen wie Cellulasen, Hemicellulasen oder/und Pektinasen zu erhalten (DE-OS 35 39 875 A1). Des weiteren sind auch mit Erfolg Versuche unternommen worden, um Komponenten wie Araban (A. E. Goodban und H. S. Owens, J. Am. Soc. Sugar Beet Technol. 9, 129 - 132, 1956) oder Pektin (F. Michel, J.-F. Thibault, C. Mercier, F. Heitz und F. Ponillande, J. of Food Sience, 50, 5, 1499 - 1502, 1985) aus dieser Gerüstsubstanz herauszulösen.

Eine weitere Möglichkeit der Verwertung besteht in der Umwandlung zu Einzellerprotein. Zahlreiche Veröffentlichungen beschäftigen sich mit dieser Möglichkeit der Veredelung von extrahierten Rübenschnitzeln. Dabei besteht der erste Schritt der Behandlung meistens in der enzymatischen Verflüssigung und Verzuckerung dieses Substrates, um dann eine Submers-Fermentation mit geeigneten Mikroorganismen, wie Hefen, durchführen zu können. Diese Prozesse ergeben schlechte Ausbeuten und sind andererseits zu unwirtschaftlich.

Eine Fermentation, in der keine freie Flüssigkeit vorhanden ist, wird als Feststoff-Fermentation bezeichnet. Solch ein Verfahren mit Preßschnitzeln als Substrat wird von P. J. Considine et al. (Biotechnology Letters Vol. 9. (2) 1987, 131 - 134) zur Herstellung von extrazellulären Hydrolasen mittels Penicillium capsulatium angewandt. W. R. Gibbons et al. (Biotechnology and Bioengeneering, Vol. 26, 1098 - 1107, 1984) beschreiben einen kontinuierlichen Feststoff-Fermentationsprozeß mit der Hefe Saccharomyces cerevisiae bei den zerkleinerte, nicht extrahierte Futterrüben zur Herstellung von Ethanol und proteinhaltigem Futter eingesetzt werden.
A. Durand et al. (European Cooperation in the field of Scientific and Technical Research - Workshop 1983, veröffentlicht von M. P. Ferranti und A. Fiedler 1983 in "Production and Feeding of Single cell proteins". Applied Science Publishers, London/New York) beschreiben ein Verfahren, bei dem extrahierte Preßschnitzel mit 20 - 22 % Trockensubstanzgehalt durch Feststoff-Fermentation mit dem Pilz Trichoderma album fermentiert werden. Das Fermentationsprodukt enthält 18 - 20 % Protein.

Aus der Literatur ist weiterhin bekannt, daß verschiedene Abfallstoffe in proteinangereicherte Fermentationsprodukte ungewandelt werden können. Dieses erfolgt vorzugsweise mittels einer Feststoff-Fermentation, wobei verschiedene niedere Pilze wie Aspergillus spezies oder Chaetomium cellulolyticum eingesetzt werden.

Andererseits beschreibt das US-Patent 4.526.791 ein Verfahren, bei dem verschiedene landwirtschaftliche Abfallstoffe mittels Chaetomium cellulotyticum in einer Submers-Fermentation zu einem proteinreichen Produkt umgewandelt werden. Der hohe Anteil an Flüssigkeit, der nach der Fermentation durch Abpressen gewonnen wird, kann gegebenenfalls zur Biogasgewinnung einer anaeroben Fermentation zugeführt werden. Der Ablauf dieses Anaerob-Reaktors, der u. a. auch anaerobe Bakterien enthält, wird sterilisiert und dann der Submers-Fermentation wieder zugeführt. Ein Teilstrom der obigen Flüssigkeit kann auch für die Vorbehandlung des einzusetzenden Feststoffs wie Stroh usw. verwendet werden. Für die Herstellung der Animpflösung wird die nach Abpressung erhaltene Flüssigkeit nicht verwendet. Überhaupt muß bei dem beschriebenen Verfahren kontinuierlich die benötigte Stickstoff- und Phosphatmenge als tierischer Kot und Urin zugegeben werden, so daß ein Überschuß an Flüssigkeit bei dem Verfahren anfällt, der entsorgt werden muß.
Aus der Literatur sind Fusarium spezies als Hydrolasenproduzenten bekannt. Danach bilden Fusarium tricinatum und -oxysporum Cellulasen und Pektinasen und eignen sich für die Herstellung solcher Enzyme. Ebenso sind von M.E.S.T. Silva und J. R. Nicoli erfolgreiche Fütterungsversuche an Ratten mit Einzellerprotein von Fusarium oxysporum var. lini (ATCC 10 960) nach einer Submers-Fermentation mit Vinasse durchgeführt worden.

Aufgabe der Erfindung ist es, ein verbessertes Verfahren zur Herstellung von proteinangereicherten Preßschnitzeln, Letztlich ist aus der EP-A-0 140 723 ein Verfahren zur Herstellung proteinreicher Nahrung aus Zuckerrüben bekannt, bei dem nach dem Prinzip der Feststoff-Fermentation z.B. Zuckerrübenbestandteile mit Mikroorganismen versetzt werden. Im einzelnen wird hier ein zuckerhaltiger Saft auf einer Unterlage aus einem Cellulose enthaltenen Material umgesetzt, wobei ein Mikroorganismus wie *Trichoderma album* eingesetzt wird, der einen hohen Anteil an Cellulasen enthält und somit zur Umsetzung der Cellulose in Zucker geeignet ist. Die aus dem Festmaterial gewonnenen Zucker werden zusammen mit den Zuckern aus der zugesetzten Lösung wie Melasse dann fermentativ durch Mikroorganismen weiterverearbeitet, deren Metaboliten sich in der Fermentationsflüssigkeit anreichern. Diese Metaboliten werden durch Abtrennen des festen und pastösen Rückstandes von der Fermentationsflüssigkeit und dadurch gewonnen, daß bei der Abtrennung die Mikroorganismen auf den festen Fermentationsrückständen zurückbleiben. Hierbei erhält man zwar insgesamt eine Anreicherung an Proteinen bei diesen Rückständen, jedoch werden die in Lösung befindlichen oder gebildeten Zuckerbestandteile bevorzugt in Citronensäure umgewandelt, so daß ein Großteil der in der Festphase enthaltenen Nährstoffe mit den abgetrennten Fermentationsbrühen verloren geht.

Aufgabe der Erfindung ist es, ein verbessertes Verfahren zur Erhöhung des Proteingehaltes in extrahierten Zuckerrübenpreßschnitzel vorzuschlagen, bei dem man mit einem Mikroorganismus eine Feststoff-Fermentation durchführt.

Die Aufgabe wird erfindungsgemäß dadurch gelöst, daß man den Trockensubstanzgehalt in der Fermentation mittels Preßsaft aus einer vorhergehenden Fermentation, dem eine geeignete Stickstoff- und Phosphatquelle sowie ein Magnesiumsalz zugefügt sind, auf 18 bis 30 % einstellt, die Fermentation bei 28 bis 31 °C während einer Zeit von 36 bis 48 Stunden durchführt, die gebildeten Schnitzel mit einem Proteingehalt von etwa 20 % durch Abpressen gewinnt, und den Preßsaft zum Einstellen des Trockensubstanzgehaltes der nächsten Fermentation verwendet.

Wesentlich ist also bei dem erfindungsgemäßen Verfahren verglichen mit dem Verfahren gemäß EP-A-0 140 723, daß die anfallende Flüssigkeit nicht wieder in den Prozeß zurückgeführt, sondern getrennt gereinigt wird und daß ferner bei der Fermentation ein bestimmter Trockensubstanzgehalt eingestellt werden muß, um eine genügend hohe Proteinkonzentration der Schnitzel zu erhalten. Arbeitet man nämlich mit einem geringen Feststoffgehalt, so ist die Proteinbildung der Mikroorganismen bezogen auf den Trockensubstanzgehalt der gewonnenen Rübenschnitzel zwar hoch, jedoch wird die Gesamtausbeute durch eine weitgehende Verstoffwechselung der enthaltenen Nährstoffe zu CO₂ und Wasser verhältnismäßig niedrig ausfallen, wobei zusätzlich noch die optimale Durchlüftung Schwierigkeiten bereitet, weil solche Lösungen mit geringer Konzentration zum Verklumpen der Feststoffe führen; arbeitet man jedoch mit höheren Feststoffkonzentrationen, sind die Wachstumsbedingungen der Mikroorganismen nicht mehr opitmal, so daß trotz einer hohen Ausbeute an Trockensubstanz der enthaltene Proteinanteil weniger hoch ist.

Weitere vorteilhafte Ausgestaltungen des erfindungsgemäßen Verfahrens sind in den Unteransprüchen aufgeführt.

Die nach dem erfindungsgemäßen Verfahren hergestellten Fermentationsprodukte sind wegen ihres erhöhten Proteingehaltes zur Verwendung als Futter für monogastrische Tiere besonders geeignet und können letztlich auch als Ausgangsprodukt zur Herstellung von Einzelproteinen dienen.

Wenn der Trockensubstandgehalt zwischen 18 bis 23 % liegt, wird ein Fermentationsprodukt mit einem hohen Proteingehalt insbesondere dann erreicht, wenn *Fungi imperfecti*, zu denen auch *Fusarium oxysporum DSM 841* zu zählen ist, verwendet werden, da diese aerob besonders gut auf extrahierten Preßschnitzeln wachsen.

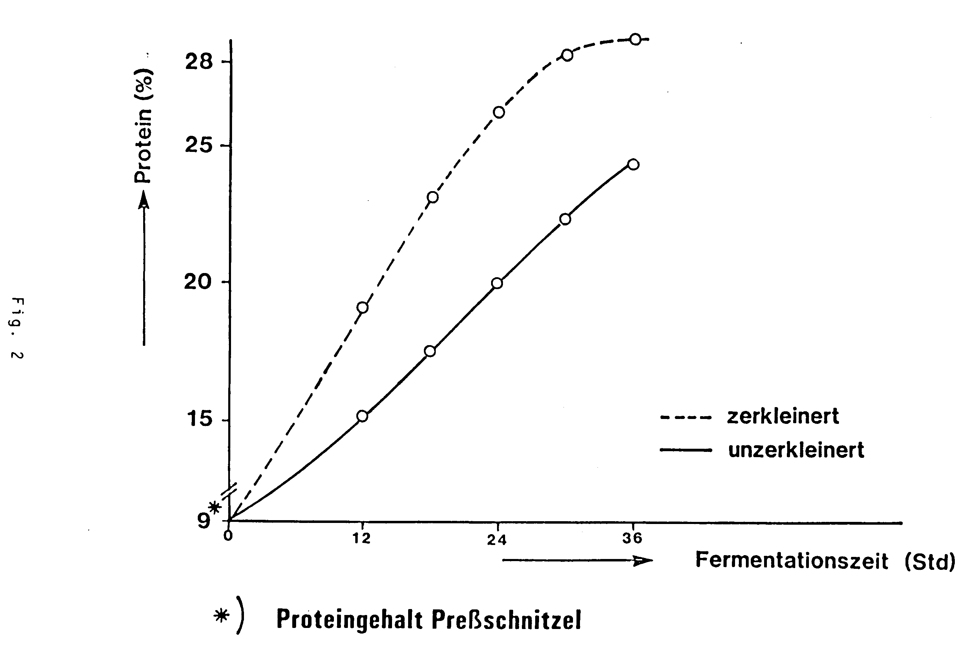

Erreicht wird bei einer auf Trockensubstand bezogenen Gewichtsausbeute von 70 bis 85 % ein Fermentationsprodukt, das einen Proteingehalt von 20 bis 28 % aufweist, wobei je nach Zerkleinerungsgrad der extrahierten Preßschnitzel etwa 36 bis 48 Stunden Fermentationszeit benötigt werden, wie es in Figur 2 gezeigt ist.

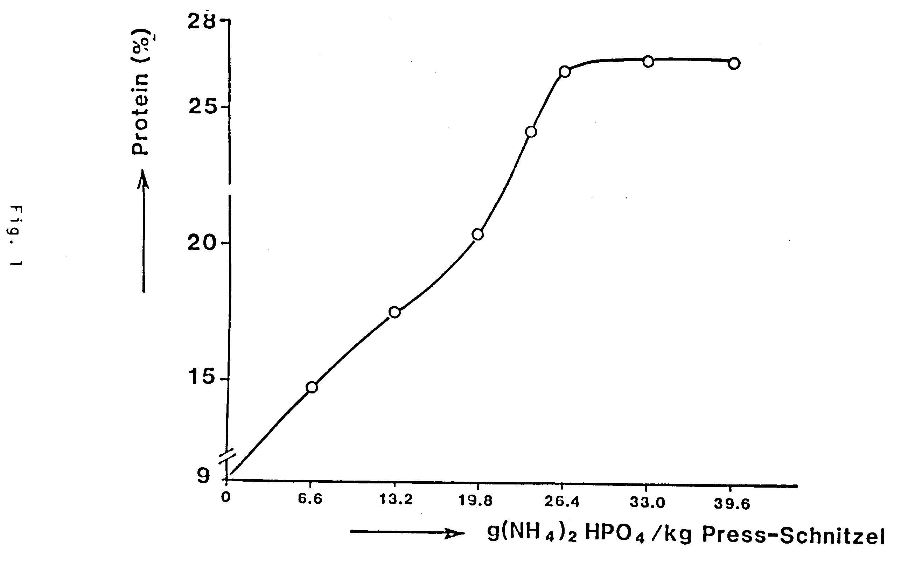

Als geeignete Stickstoff-/Phosphatquelle hat sich Diammoniumhydrogenphosphat erwiesen, das bei einer Konzentration von 26 - 40 g dieses Salzes pro kg extrahierter Preßschnitzel (30 % Trockensubstanzgehalt) die maximalen Proteinwerte bei hoher Ausbeute ergibt (Abb. 1). Anstelle von kristallinem Diammoniumhydrogenphosphat kann auch der in der Landwirtschaft verwendete DAP-(Diammoniumphosphat) Dünger eingesetzt werden. In diesem Fall werden 28 - 41 g dieses Salzes pro kg extrahierte Preßschnitzel (30 % Trockensubstanz) benötigt.

Die proteinreichen Fermentationsprodukte ergaben bei Fütterungsversuchen an Ratten eine gute Verträglichkeit, was als Beweis für das Nichtvorhandensein von Toxinen im Fermentationsprodukt gelten kann.
Weiterhin wurde Überraschenderweise gefunden, daß durch Abpressen des Fermentationsproduktes ein Preßsaft erhalten wird, der nach einer Pasteurisierung für 10 - 15 Minuten bei 90 - 95 °C, einerseits zur Herstellung der benötigten Animpflösung verwendet werden kann, andererseits anstatt von Wasser zur Einstellung des gewünschten Trockensubstanzgehaltes der zu fermentierenden Preßlinge von 18 - 23 % zugegeben wird.

Daraus ergeben sich zu bisher bekannten Fermentationsprozessen, die bei hohem Trockensubstanzgehalten geführt werden, entscheidende Vorteile:
Das Abpressen des Fermentationsproduktes erhöht den Trockensubstanzgehalt des Produktes, womit ein geringerer Energiebedarf für die Trocknung notwendig ist, um zu einem lagerfähigen Produkt zu gelangen.

Die Rückführung der flüssigen Phase bedeutet überdies, daß der Frischwasserbedarf auf ein Minimum verringert wird und kein stark belastetes Abwasser anfällt.

Vor allem aber kann die Gesamtmenge an Stickstoff-/Phosphat-Salz reduziert werden, da der zurückgenommene Preßsaft überflüssige Anteile dieses Nährsalzes enthält und diese wieder in den Fermentationsprozeß zurückgeführt werden.

Lösliche Oligo- und Polysaccharide, die der Gerüstsubstanz der Rüben entstammen, dienen als Kohlenstoffquelle einerseits und wirken gleichzeitig induzierend auf die Neusynthese von Hydrolasen wie Cellulasen, Hemicellulasen und Pektinasen beim Mikroorganismus. Daraus resultiert eine Bildung dieser für den Abbau der Preßschnitzel nötigen Hydrolasen in der Animpfunglösung. Für den eigentlichen Fermentationsprozeß ergibt sich daraus ein gutes und schnelles Wachstum auf dem angebotenen Substrat. Dieses führt zur Reduzierung der gesamten Fermentationszeit.

Durch Verwendung von Fusarium oxysporum DSM 841 wird vorzugsweise das Pektin neben der Hemicellulose abgebaut. Dieses weitgehend vom Pektin befreite Fermentationsprodukt läßt sich leicht mechanisch entwässern.

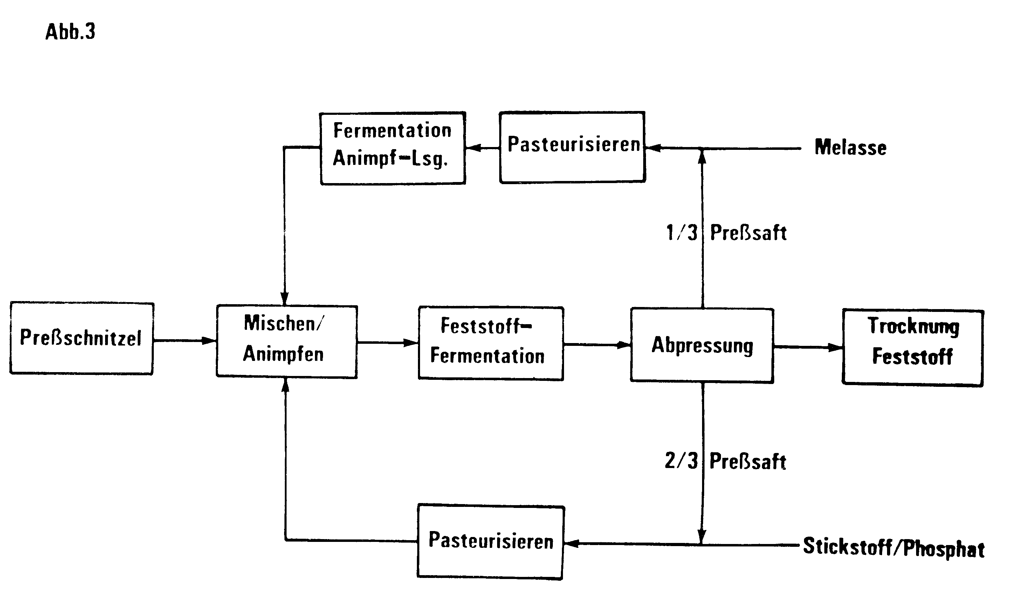

Für das erfindungsgemäße Verfahren ergibt sich somit das in Abbildung 3 aufgezeigte Fließschema für einen kontinuierlichen Fermentationsprozeß. Danach werden die extrahierten Rübenpreßschnitzel nur beim Anfahren des Fermentationsprozesses mit Frischwasser auf 18 - 23 % Trockensubstanzgehalt eingestellt. Nach dem ersten Fermentationsprozeß wird das Fermentationsprodukt soweit abgepreßt, wie nötig ist, um die Animpflösung erstellen zu können und um weiterhin zusammen mit der Animpfung den erforderlichen Trockensubstanzgehalt von 18 - 23 % bei frischzugeführten Rübenpreßschnitzeln einstellen zu können. Beides wird vor Wiederverwendung für 10 - 15 Minuten bei 90 - 95 °C pasteurisiert. Dabei wird so verfahren, daß für die Animpflösung ein Drittel des Preßsaftes eingesetzt wird und die verbliebenen zwei Drittel nach Zugabe des Stickstoff-/Phosphatsalzes zur Einstellung des gewünschten Trockensubstanzgehaltes benötigt werden.

Zur Animpfung werden vor der Pasteurisierung geringe Mengen an Rübenmelasse zugegeben.

Die Erfindung wird durch folgende Beispiele näher erläutert:

### Beispiel 1 a

In einem 300-ml-Erlenmeyerkolben werden zu 100 ml Wasser 1,5 g Rübenmelasse und 0,5 g gemahlene Trockenschnitzel gegeben. Mit Natronlauge wird der pH-Wert auf 4,7 eingestellt. Nach einer Pasteurisierung des Ansatzes für 15 Minuten bei 90 °C wird mit einer sporen- und myzelhaltigen Pilzsuspension von Fusarium oxysporum DSM 841 angeimpft.
Der Mikroorganismus ist dazu zuvor auf einem Kartoffel-Glucose-Agar für 5 Tage bei 28 °C angezüchtet worden. Die Animpflösung wird durch Ablösen des Pilzes mit 10 ml sterilem Wasser gewonnen. 2 ml dieser Lösung werden zum Animpfen des obigen Ansatzes verwendet.
Nach einer Anzüchtungsphase von 24 - 72 Stunden bei 28 °C und unter aeroben Bedingungen können mit diesem Ansatz 500 g extrahierte Rübenschnitzel (30 % Trockensubstanzgehalt) für eine Feststoff-Fermentation angeimpft werden.

### Beispiel 1 b

Wie Beispiel 1 a jedoch wird anstelle von Wasser bei der Herstellung der Animpflösung 100 ml Preßsaft eingesetzt. Letzterer wird erhalten, indem nach einer Feststoffermentation von extrahierten Preßschnitzeln mit Fusarium oxysporum DSM 841 das erhaltene Fermentationsprodukt abgepreßt wird. Die gewonnene flüssige Phase, hier als Preßsaft bezeichnet, wird nach Zugabe von 0,75 g Rübenmelasse ebenfalls einer Pasteurisierung für 15 Minuten bei 90 °C unterzogen und zur Herstellung der Animpflösung verwendet.

Auch hier ergeben Animpflösungen mit einer Anzuchtzeit von 24 - 72 Stunden bei der späteren Feststoffermentation gleich gutes Wachstum.

### Beispiel 2

In einen 1-Liter-Erlenmeyerkolben werden 400 ml Preßsaft und 3 g Rübenmelasse gegeben. Die Lösung wird für 15 Minuten bei 90 °C pasteurisiert. Nach dem Abkühlen erfolgt die Zugabe von 8 ml Animpflösung, die wie in Beispiel 1 a beschrieben, hergestellt wird. Die Fermentation wird unter Rühren und zusätzlicher Luftbegasung bei 30 °C für 24 h durchgeführt.

Danach wird täglich soviel myzelhaltige Lösungen kontinuierlich entnommen gleichzeitig der gleiche Volumenanteil an Preßsaft zugeführt, daß sich eine mittlere Aufenthaltszeit von 24 Stunden ergibt. Diese Fahrweise ist über 9 Tage beibehalten worden.
Täglich sind 10 ml der abgenommenen myzelhaltigen Lösung zum Animpfen von 50 g extrahierten Preßschnitzeln (30 % Trockensubstanzgehalt) eingesetzt worden. Dieser wurden zuvor 20 ml einer Lösung, die 1,32 g (NH₄)₂HPO₄ und 0,125 g MgSO₄ enthielt, zugegeben.
In allen Fällen erfolgte ein gutes Wachstum von Fusarium oxysporum DSM 841.

### Beispiel 3

Je 100 g extrahierte Preßschnitzel (30 % Trockensubstanzgehalt) werden in einem 500-ml-Erlenmeyerkolben mit 40 ml einer Lösung vermischt, die jeweils 0,25 g MgSO₄ und unterschiedliche Mengen an (NH₄)₂HPO₄(0,66; 1,32; 1,98; 2,64; 3,30; 3,96 g) enthält. Angeimpft wird mit Fusarium oxysporum DSM 841. Herstellung der Animpflösung wie in Beispiel 1 a. Die Fermentationen erfolgten bei 30 °C für 48 Stunden.

Die erhaltenen Proteingehalte der von löslichen Bestandteilen befreiten Rückstände in Abhängigkeit von der Konzentration an (NH₄)₂HPO₄ sind in Abbildung 1 aufgezeigt.

### Beispiel 4

Wie Beispiel 3, anstelle von kristallinem (NH₄)₂HPO₄ sind unterschiedliche Mengen an DAP (Diammoniumphosphat)-Dünger eingesetzt worden.
Die Proteingehalte liegen zwischen 20 und 24 %.

### Beispiel 5

In fünf 500 ml Erlenmeyerkolben werden je 100 g extrahiertes und zerkleinertes (3-4 mm) Preßschnitzelmaterial (30 % Trockensubstanzgehalte) mit 40 ml einer Lösung vermischt, die 0,25 g MgSO₄ und 1,32 g(NH₄)₂HPO₄ enthält. Animpfung erfolgt wie in Beispiel 1 beschrieben. Fermentiert wird bei 30 °C für 12, 18, 24, 30 und 36 Stunden. Als Kontrolle werden unter gleichen Bedingungen 5 mal je 100 g extrahierte Preßschnitzel eingesetzt.
Die Proteingehalte der von löslichen Bestandteilen befreiten Rückstände sind in Abbildung 2 dargestellt worden.

### Beispiel 6

700 g extrahierte Preßschnitzel (30 % Trockensubstanzgehalte) werden mit 280 ml einer Lösung, die 21 g(NH₄)₂HPO₄ und 1,75 MgSO₄ enthält, vermischt, bevor 140 ml Animpflösung (Beispiel 1) zugegeben werden. Das Material wird zu einer Schichtdicke von 3 - 4 cm in einem Kunststoffwanne lose verteilt und bei einer relativen Luftfeuchtigkeit um 99 % bei 30 °C für 48 h fermentiert. Das fermentierte Produkt wird abgepreßt, so daß 420 ml Preßsaft erhalten werden.
Zu einem Drittel dieser Lösung wird 1 g Melasse gegeben. Es folgt eine Pasteurisierung der Lösung für 15 Minuten bei 90 °C, bevor mit einer sporen- und myzelhaltigen Lösung von Fusarium oxysporum DSM 841 angeimpft wird. Die 24 Stunden alte Animpflösung wird dann für die folgende Feststoffermentation eingesetzt.
Zu zwei Drittel des Preßsaftes werden 18,5 g (NH₄)₂HPO₄ gegeben. Nach Pasteurisierung werden 700 g extrahierte Preßschnitzel mit dieser Lösung vermischt, bevor die zuvor angeführte Animpflösung zugegeben wird.
Die Fermentation ist mehr als 10 mal wiederholt worden. In allen Fällen wird ein gutes Wachstum von Fusarium oxysporum DSM 841 erreicht. Die Proteingehalte betragen zwischen 20 - 23 % auf Trockensubstanzgehalt.

### Beispiel 7

Wie Beispiel 6, anstelle von (NH₄)₂HPO₄ wird 24 g DAP-Dünger eingesetzt.
In allen Fällen ist gutes Wachstum zu beobachten und die Proteingehalte liegen bei 20 - 23 % auf Trockensubstanzgehalt.

## Patentansprüche

1. Verfahren zur Erhöhung des Proteingehaltes in extrahierten Zuckerrübenpreßschnitzeln, bei dem man mit einem Mikroorganismus eine Feststoff-Fermentation durchführt, **dadurch gekennzeichnet**, daß man den Trockensubstanzgehalt in der Fermentation mittels Preßsaft aus einer vorhergehenden Fermentation, dem eine geeignete Stickstoff- und Phosphatquelle, sowie ein Magnesiumsalz zugefügt sind, auf 18 bis 30 % einstellt, die Fermentation bei 28 bis 31 °C während einer Zeit von 36 bis 48 Stunden durchführt, die gebildeten Schnitzel mit einem Proteingehalt von ca. 20 % durch Abpressen gewinnt, und den Preßsaft zum Einstellen des Trockensubstanzgehaltes der nächsten Fermentation verwendet.

2. Verfahren gemäß Anspruch 1, **dadurch gekennzeichnet**, daß man die Fermentation mit auf 18 bis 23 % Trockensubstanzgehalt eingestellten Preßschnitzeln durchführt.

3. Verfahren gemäß Anspruch 1 oder 2, **dadurch gekennzeichnet**, daß man den Preßsaft der Fermentation nach einer Pasteurisierung für 10 bis 15 Minuten bei 90 bis 95 °C zur Herstellung der benötigten Animpflösung verwendet.

4. Verfahren gemäß den Ansprüchen 1 bis 3,
dadurch gekennzeichnet,
daß man die Fermentation bei hoher Luftfeuchtigkeit durchführt.

5. Verfahren gemäß Anspruch 4,
dadurch gekennzeichnet,
daß man die Fermentation bei 99 % Luftfeuchtigkeit durchführt.

6. Verfahren gemäß den Ansprüchen 1 bis 5,
dadurch gekennzeichnet,
daß man als Mikroorganismus den Stamm Fusarium oxysporum DSM 841 einsetzt.

7. Verfahren gemäß den Ansprüchen 1 bis 6,
dadurch gekennzeichnet,
daß man als Stickstoff- und Phosphorquelle für die Fermentation Diammoniumhydrogenphosphat, vorzugsweise Diammoniumphosphat-Dünger, verwendet.

8. Verfahren gemäß Anspruch 7,
dadurch gekennzeichnet,
daß 26 bis 40 g, vorzugsweise 33 g Diammoniumhydrogenphosphat pro kg extrahierter Preßschnitzel (30 % Trockensubstanzgehalt) eingesetzt werden.

9. Verfahren gemäß Anspruch 7,
dadurch gekennzeichnet,
daß 28 bis 41 g Diammoniumphosphat-Dünger pro kg extrahierter Preßschnitzel (30 % Trockensubstanzgehalt) eingesetzt werden.

10. Verfahren gemäß den Ansprüchen 1 bis 8,
dadurch gekennzeichnet,
daß der Fermentationsprozeß kontinuierlich betrieben wird.

11. Verwendung der gemäß den Ansprüchen 1 bis 6 hergestellten Fermentationsprodukten als Futter für monogastrische Tiere.

## Claims

1. Method for increasing the protein content in extracted sugar beet pressed chips, wherein a solids-fermentation is carried out with a micro-organism, characterised in that the dry substance content in the fermentation is adjusted to 18 to 30 % by means of pressed juice from a previous fermentation, to which pressed juice a suitable nitrogen and phosphate source and also a magnesium salt are added, the fermentation is carried out at 28 to 31°C for a period of 36 to 48 hours, the chips which are formed, having a protein content of approximately 20%, are obtained by being pressed out and the pressed juice is used to adjust the dry substance content of the next fermentation.

2. Method according to claim 1, characterised in that the fermentation is carried out with pressed chips which are adjusted to a dry substance content of 18 to 23%.

3. Method according to claim 1 or 2, characterised in that the pressed juice of the fermentation is used, after pasteurization for 10 to 15 minutes at 90 to 95°C, for the preparation of the required inoculation solution.

4. Method according to claims 1 to 3, characterised in that the fermentation is carried out at high humidity of air.

5. Method according to claim 4, characterised in that the fermentation is carried out at 99% humidity of air.

6. Method according to claims 1 to 5, characterised in that the strain Fusarium oxysporum DSM 841 is used as the micro-organism.

7. Method according to claims 1 to 6, characterised in that diammonium hydrogen phosphate, preferably diammonium phosphate-fertilizer, is used as the nitrogen and phosphorus source for the fermentation.

8. Method according to claim 7, characterised in that 26 to 40 g, preferably 33 g diammonium hydrogen phosphate, are used per kg of extracted pressed chips (30% dry substance content).

9. Method according to claim 7, characterised in that 28 to 41 g diammonium phosphate-fertilizer are used per kg of extracted pressed chips (30% dry substance content).

10. Method according to claims 1 to 8, characterised in that the fermentation process is run continuously.

11. Use of the fermentation products prepared according to claims 1 to 6 as feed for monogastric animals.

## Revendications

1. Procédé pour l'augmentation de la teneur en protéines de gâteaux pressés de betterave, dans lequel on effectue une fermentation de matières solides au moyen d'un microorganisme, caractérisé en ce qu'on ajuste à une valeur de 18% à 30% la teneur en matières solides pendant la fermentation à l'aide de jus pressé d'une fermentation précédente, auquel jus a été ajoutée une source propre d'azote et de phosphate, telle qu'un sel de magnésium, en ce que la fermentation est effectuée à une température de 28 à 31°C pendant une durée de 36 à 48 heures, en ce que les gâteaux formés avec une teneur en protéines d'environ 20% sont obtenus par extraction par pression, et en ce que le jus pressé est utilisé pour ajuster la teneur en matières solides pendant la fermentation suivante.

2. Procédé suivant la revendication 1, caractérisé en ce que la fermentation est effectuée sur des gâteaux pressés ajustés à une teneur en matières solides de 18 à 23%.

3. Procédé suivant la revendication 1 ou 2, caractérisé en ce qu'on utilise le jus pressé de la fermentation, après une pasteurisation pendant 10 à 15 minutes à une température de 90 à 95°C, pour la production de la solution d'inoculation nécessaire.

4. Procédé suivant les revendications 1 à 3, caractérisé en ce que la fermentation est effectuée dans une humidité atmosphérique élevée.

5. Procédé suivant la revendication 4, caractérisé en ce que la fermentation est effectuée à une humidité atmosphérique de 99% .

6. Procédé suivant les revendications 1 à 5, caractérisé en ce que la souche Fusarium oxysporum DSM 841 est utilisée comme microorganisme.

7. Procédé suivant les revendications 1 à 6, caractérisé en ce qu'un diammoniumhydrogênephosphate, de préférence un engrais de diammoniumphosphate, est utilisé comme source d'azote et de phosphore.

8. Procédé suivant la revendication 7, caractérisé en ce qu'on utilise de 26 à 40 g, de préférence 33 g, de diammoniumhydrogênephosphate par kg de gâteau extrait (teneur en matières solides 30%).

9. Procédé suivant la revendication 7, caractérisé en ce qu'on utilise de 28 à 41 g d'engrais de diammoniumphosphate par kg de gâteau extrait (teneur en matières solides 30%).

10. Procédé suivant les revendications 1 à 8, caractérisé en ce que la fermentation est effectuée en continu.

11. Utilisation des produits fermentés obtenus suivant les revendications 1 à 6 comme aliments pour des animaux à estomac simple.
